# EUROPEAN PATENT APPLICATION

(11) **EP 4 722 158 A1**
(43) Date of publication of application: **08.04.2026**
(21) Application number: 24815652.3
(22) Date of filing: 08.02.2024
(51) Int. Cl.: C01B 33/18, C01B 33/14, A61K 8/02, A61K 8/25, A61K 8/31, A61K 9/00, A61K 47/02, A61Q 7/00, A61Q 9/00, A61Q 19/00

(54) **HOLLOW MESOPOROUS SILICA ROD, METHOD FOR PREPARING SAME, AND METHOD FOR SUPPORTING ACTIVE SUBSTANCE**

(30) Priority: 30.05.2023 KR 20230069011
(71) Applicant: BN Co., Ltd., Gyeonggi-do 14059 (KR); Pusan National University Industry-University Cooperation Foundation, Busan 46241 (KR)
(72) Inventor: KIM, Jaehyuk, Busan 46296 (KR); KWON, Tae Gwang, Seoul 05847 (KR); JEON, Moo Seong, Anyang-si Gyeonggi-do 14102 (KR); KIM, Du Jin, Yongin-si Gyeonggi-do 16899 (KR); CHOI, Hak Lyul, Yongin-si Gyeonggi-do 16860 (KR); JUNG, Hyun Ho, Yongin-si Gyeonggi-do 16941 (KR)
(74) Representative: Pons IP
(86) International application number: PCT/KR2024/001931
(87) International publication number: WO 2024/248269

(57) **Abstract**

The present invention provides a hollow mesoporous silica rod, a method for producing the same, and a method for supporting an active substance. The hollow mesoporous silica rod of the present invention has a wide inner hollow portion and a large aspect ratio, can stably support a large amount of active substance, and can penetrate the surface of the skin to easily reach a predetermined depth inside the skin, thereby effectively delivering the active substance.

## Description

### Technical Field

The present invention relates to a hollow mesoporous silica rod and a method for producing the same. Particularly, the present invention relates to a silica rod capable of supporting an active substance within the hollow portion and a method for producing the same.

### Background Art

Silica refers to silicon dioxide (SiO₂) and is known by various names depending on its size and shape. Silica is inexpensive in terms of raw materials, can be produced into highly uniform particles using a simple synthesis method, and is a biocompatible substance with low toxicity, so it is utilized in various fields such as catalysts, materials, fibers, and agriculture. The Stöber process is the most representative silica synthesis method, capable of synthesizing uniform silica particles of various sizes. The Stöber process synthesizes silica through a chain reaction of hydrolysis and condensation of a silica precursor using an alcohol/water mixture as a solvent and ammonia as a catalyst. Silica typically grows in a spherical shape. Rather than using it directly, it is produced into a desired shape through additional reactions and surface modifications as needed.

Mesoporous silica has mesopores having a size of 2 nm to 50 nm on its surface, and these mesopores significantly increase its specific surface area. In order to produce mesoporous silica, a silica precursor and a pore-directing agent that later forms mesopores are required. Polymers, surfactants, and the like, are used as pore-directing agents. Pore-directing agents dispersed in a mixture of water and alcohol form spherical micelles, which are then stacked layer by layer to form a stabilized cylindrical shape. The polymerization reaction of the silica precursor causes silica to grow on the surface of the micelle cylinders. Upon removal of the pore-directing agent, the pores become mesopores, resulting in mesoporous silica.

For example, Korean Patent Application Publication No. 10-2006-0129824 discloses a technique for capturing L-menthol in mesoporous silica produced in this manner. However, the type, amount, and ease of use of the active ingredient that can be accommodated are limited. Therefore, research and development of mesoporous silica materials that can easily accommodate a variety of active substances in large quantities are needed.

### Detailed Description of Invention

### Technical Problem

One object of the present invention is to provide a hollow mesoporous silica rod.

One object of the present invention is to provide a method for producing a hollow mesoporous silica rod.

One object of the present invention is to provide a method for supporting an active substance within a hollow mesoporous silica rod.

One object of the present invention is to provide a cosmetic composition comprising a hollow mesoporous silica rod.

One object of the present invention is to provide a pharmaceutical composition comprising a hollow mesoporous silica rod.

### Solution to Problem

In one embodiment of the present invention, there is provided a hollow mesoporous silica rod comprising: a mesoporous silica shell; and a hollow portion within the silica shell, wherein the silica shell is rod-shaped and has an aspect ratio of 1:2 to 1:100.

In some embodiments, the silica shell may have a thickness of 5 nm to 100 nm.

In some embodiments, the volume of the hollow portion may be 50% to 99% based on the volume defined by the outer surface of the silica shell.

In some embodiments, the volume of the hollow portion may be 10 times or more of the total volume of the mesopores included in the mesoporous silica shell.

In some embodiments, the silica shell may be formed of organic silica.

In some embodiments, it may further comprise an active substance supported within the hollow portion.

In some embodiments, the active substance may comprise a hydrophobic substance.

In some embodiments, the active substance may include any one selected from mineral oil, oleic acid, methyl oleate, vegetable oil, caprylic/capric triglyceride, ascorbyl tetraisopalmitate, oil-soluble licorice extract, alpha-bisabolol, retinyl palmitate, tocopherol, madeca, or terpenes.

In some embodiments, it may further comprise a capping layer that caps the mesoporous pores of the silica shell.

In some embodiments, the capping layer may be formed of inorganic silica.

In some embodiments, the silica shell may have an aspect ratio of 1:2 to 1:40.

In one embodiment of the present invention, there is provided a method for producing a hollow mesoporous silica rod, comprising the steps of: preparing a rod-shaped inorganic silica core; forming a mesoporous silica shell on the surface of the inorganic silica core to produce a core-shell silica rod; and removing the inorganic silica core within the core-shell silica rod.

In some embodiments, the step of preparing a rod-shaped inorganic silica core may comprise the steps of: preparing an oil-in-water emulsion comprising an alcoholic solvent and a surfactant; and adding an inorganic silica precursor to the oil-in-water emulsion to grow the inorganic silica in a rod shape.

In some embodiments, the step of preparing a rod-shaped inorganic silica core may comprise the steps of: preparing an inorganic silica nanoseed by adding a silica precursor to a solution comprising an alcoholic solvent and a silica-forming catalyst; preparing an oil-in-water emulsion comprising the inorganic silica nanoseed, an alcoholic solvent, and a surfactant; and adding an inorganic silica precursor to the oil-in-water emulsion to grow the inorganic silica in a rod shape.

In some embodiments, the mesoporous silica shell may be formed by reacting the inorganic silica core with a surfactant and an organic silica precursor in a mixed solvent of water and an alcoholic solvent.

In some embodiments, the step of removing the inorganic silica core may comprise etching the core-shell silica rod with an alkaline solution.

In some embodiments, the alkaline solution may comprise an alkali metal hydroxide.

In some embodiments, the reaction temperature of the alkaline solution may be 50°C to 90°C.

In one embodiment of the present invention, there is provided a method for supporting an active substance, comprising the steps of: mixing the hollow mesoporous silica rod with an active substance; and injecting the active substance into the hollow portion of the hollow mesoporous silica rod.

In some embodiments, the active substance may comprise a hydrophobic substance, and the step of injecting the active substance may comprise forming an oil-in-water emulsion from a mixed solution of the hollow mesoporous silica rod and the active substance.

In some embodiments, the method for supporting an active substance may further comprise the step of removing any remaining active substance not injected into the hollow portion by centrifugation.

In some embodiments, the method for supporting an active substance may further comprise the step of capping mesoporous pores present in the shell of the silica rod into which the active substance has been injected.

In one embodiment of the present invention, there is provided a cosmetic composition comprising the hollow mesoporous silica rod, wherein the active substance has been supported within the hollow portion, as an active ingredient.

In some embodiments, the cosmetic composition may be used for whitening, ameliorating wrinkles, removing body hair, alleviating acne-prone skin, alleviating hair loss symptoms, enhancing elasticity, or moisturizing the skin.

In one embodiment of the present invention, there is provided a pharmaceutical composition comprising the hollow mesoporous silica rod, wherein the active substance has been supported within the hollow portion, as an active ingredient.

### Effects of Invention

The hollow mesoporous silica rod of the present invention can easily support a large amount of active substance through its inner hollow portion and surface mesopores, and can stably maintain the supported active substance within the rod. In addition, its large aspect ratio allows it to easily penetrate the surface of the skin, effectively delivering the supported active substance to a predetermined depth. Furthermore, its anisotropy allows the particles to be aligned, facilitating the entry and exit of the active substance in a specific direction. Therefore, the hollow mesoporous silica rod of the present invention can effectively deliver a large amount of active substance to the epidermal layer.

The hollow mesoporous silica rod of the present invention can be produced economically and efficiently using readily available materials and a simple process.

### Brief Description of Drawings

Figure 1 is a schematic flowchart showing the growth process of an inorganic silica rod.
Figure 2 is a scanning electron microscope (SEM) image of inorganic silica rods produced according to some embodiments.
Figure 3 is a transmission electron microscope (TEM) image of inorganic silica rods produced according to some embodiments.
Figure 4 is an optical microscope and scanning electron microscope (SEM) image of inorganic silica rods produced according to some embodiments.
Figure 5 is a schematic flowchart showing the production process of a hollow mesoporous silica rod.
Figure 6 is a transmission electron microscope (TEM) image of hollow mesoporous silica rods produced based on the inorganic silica rods shown in Figure 2.
Figure 7 is an optical microscope and scanning electron microscope (SEM) image of hollow mesoporous silica rods produced based on the inorganic silica rods shown in Figure 2.
Figure 8 is a schematic flowchart showing the process of supporting an active substance onto a hollow mesoporous silica rod.
Figure 9 is a transmission electron microscope image of hollow mesoporous silica rods before (left) and after (right) supporting squalene.
Figure 10 is a photograph and optical microscope image of hollow mesoporous silica rods before and after supporting madeca.
Figure 11 is a transmission electron microscope image of hollow mesoporous silica rods before (left) and after (right) supporting squalene.
Figure 12 is an optical microscope image and a fluorescence microscope image and a photograph of hollow mesoporous silica rods and fluorescently labeled hollow mesoporous silica rods.
Figure 13 is a fluorescence microscope image of fluorescently labeled active substance-supported hollow mesoporous silica rods.
Figure 14 is a graph of red and green fluorescence intensities for the same cross-section of the fluorescently labeled active substance-supported hollow mesoporous silica rods.
Figure 15 is an optical microscope image and a fluorescence microscope image comparing the degree of penetration of the active substance in a group treated with an example sample, a group treated with an active substance alone, and a control group in the research pig skin.
Figure 16 is a graph showing the fluorescence intensity within the epidermis, excluding the stratum corneum, of the fluorescent image of the active substance in Figure 15.

### Mode for Carrying out the Invention

In one aspect of the present invention, there is provided a hollow mesoporous silica rod. The hollow mesoporous silica rod may comprise a mesoporous silica shell; and a hollow portion within the silica shell, wherein the silica shell may be rod-shaped and have an aspect ratio of 1:2 to 1:100.

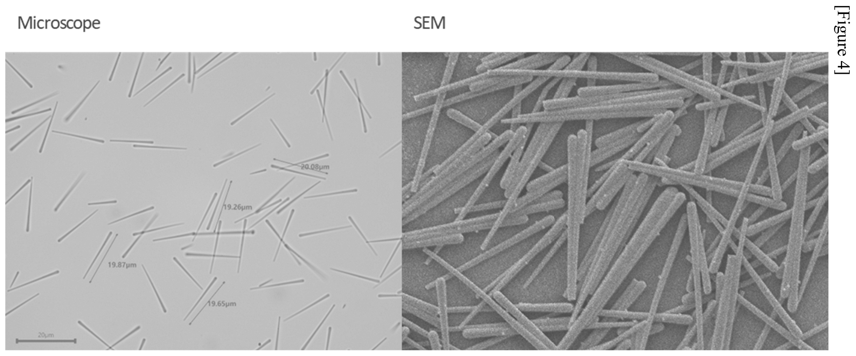

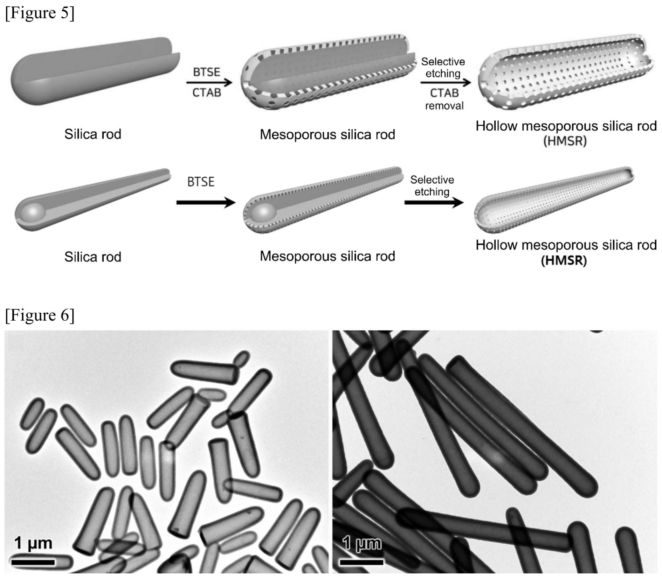

Specifically, referring to Figures 4 and 6, a hollow mesoporous silica rod comprises a mesoporous silica shell and a hollow portion within the silica shell.

In some embodiments, the silica shell may be an organic silica shell. For example, the silica shell may be formed of organic silica.

Organic silica is silica that further comprises organic groups such as carbon and hydrogen in addition to the silicon (Si) and oxygen (O) elements that constitute typical silica (SiO₂). For example, organic silica comprises hydrocarbon groups remaining from the polymerization reaction of an organic alkoxysilane in which a silicon central atom is substituted with a hydrocarbon group in addition to alkoxy groups. In particular, organic silica with residual alkylene groups can be formed by the polymerization reaction of an organic silica precursor, such as an alkoxysilylalkylene, in which two or more alkoxysilyl groups are linked by an alkylene group.

Mesoporous organic silica is silica in which numerous mesopores with a size (diameter) of 2 nm to 50 nm are formed by self-assembly of a surfactant. The pore size is controlled by the type of surfactant and the additive (such as trimethylbenzene).

The term "shell" refers to a member that encloses an internal material or space and separates the interior from the exterior. The shell may have an external surface facing outward, an internal surface facing inward, and a predetermined thickness defined by the distance between the external and internal surfaces.

The silica shell is rod-shaped and has an aspect ratio of 1:2 to 1:100. When the aspect ratio is less than 1:2, a sufficient amount of active substance cannot be supported within a hollow mesoporous silica rod, and it may be difficult to deliver the active substance to the target depth. When the aspect ratio is more than 1:100, it may be difficult for the active substance to pass through the silica shell and be supported into the hollow portion. For example, the aspect ratio may be 1:2 to 1:90, 1:2 to 1:80, 1:2 to 1:70, 1:2 to 1:60, 1:2 to 1:50, 1:2 to 1:40, 1:2 to 1:30, 1:2 to 1:20, 1:3 to 1:100, 1:3 to 1:90, 1:3 to 1:80, 1:3 to 1:70, 1:3 to 1:60, 1:3 to 1:50, 1:3 to 1:40, 1:3 to 1:30, 1:3 to 1:20, 1:4 to 1:100, 1:4 to 1:90, 1:4 to 1:80, 1:4 to 1:70, 1:4 to 1:60, 1:4 to 1:50, 1:4 to 1:40, 1:4 to 1:30, 1:4 to 1:20, 1:5 to 1:100, 1:5 to 1:90, 1:5 to 1:80, 1:5 to 1:70, 1:5 to 1:60, 1:5 to 1:50, 1:5 to 1:40, 1:5 to 1:30, or 1:5 to 1:20.

The term "rod shape" refers to a shape having a first minor axis, a second minor axis, and a major axis in increasing order of length, wherein the length of the first minor axis is similar to the length of the second minor axis, and the major axis is longer than the length of the first minor axis and the length of the second minor axis, and has no vertices. For example, the rod shape may include a cylinder, an ellipsoid, a capsule, and the like.

The term "aspect ratio" refers to the ratio of the length of the major axis to the average length of the first minor axis and the second minor axis.

In some embodiments, the silica shell may have a thickness of 5 nm to 100 nm. Preferably, the silica shell may have a thickness of 5 nm to 80 nm, 5 nm to 60 nm, 5 nm to 50 nm, 5 nm to 40 nm, 10 nm to 100 nm, 10 nm to 80 nm, 10 nm to 60 nm, 10 nm to 50 nm, 10 nm to 40 nm, 20 nm to 100 nm, 20 nm to 80 nm, 20 nm to 60 nm, 20 nm to 50 nm, or 20 nm to 40 nm. In this case, the active substance can be easily supported within the hollow portion, the supported active substance can remain stable without being released, and the silica shell can be easily degraded in the target environment, thereby effectively delivering the active substance.

The length of the hollow mesoporous silica rod (the length of the major axis) may be 10 µm or greater. For example, the length of the hollow mesoporous silica rod may be 12 µm or greater, 15 µm or greater, or 18 µm or greater. In this case, the hollow mesoporous silica rod can penetrate the stratum corneum and reach the epidermal layer. At this time, the active substance supported within the hollow mesoporous silica rod can be delivered into the epidermis. In addition, the length of the hollow mesoporous silica rod may be 100 µm or less.

The thickness of the hollow mesoporous silica rod (the length of the first minor axis or the second minor axis or the diameter) may be 5 µm or less. For example, the thickness of the hollow mesoporous silica rod may be 4 µm or less, 3 µm or less, or 2 µm or less. In this case, the hollow mesoporous silica rod can reach the epidermal layer without breaking while penetrating the stratum corneum, and can support a suitable amount of an active substance within it. This allows a large amount of an active substance to be delivered into the epidermis. In addition, the thickness of the hollow mesoporous silica rod may be 0.1 µm or greater.

The silica shell may have mesopores which penetrate along its thickness. Alternatively, a plurality of mesopores may be interconnected to form channels, which penetrate the silica shell along its thickness.

The "hollow portion" may refer to an empty space surrounded by the inner surface of the silica shell. An active substance may be supported into the hollow portion.

The hollow portion may be in communication with the outside through the mesopores of the silica shell. The active substance may be supported within the hollow portion through the mesopores.

In some embodiments, the volume of the hollow portion may be 50% to 99% based on the volume defined by the outer surface of the silica shell. Preferably, the volume of the hollow portion may be 50% to 98%, 50% to 97%, 50% to 96%, 50% to 95%, 50% to 90%, 50% to 85%, 50% to 80%, 50% to 75%, 50% to 70%, 50% to 65%, 50% to 60%, 60% to 99%, 60% to 98%, 60% to 97%, 60% to 96%, 60% to 95%, 60% to 90%, 60% to 85%, 60% to 80%, 60% to 75%, 60% to 70%, or 60% to 65% based on the volume defined by the outer surface of the silica shell. In this case, a large amount of the active substance can be easily supported within the hollow mesoporous silica rod, and leakage of the supported substance can be prevented.

In some embodiments, the volume of the hollow portion may be 10 times or more of the total volume of the mesopores included in the mesoporous silica shell. Preferably, the volume of the hollow portion may be 12 times or more, 15 times or more, 20 times or more, 25 times or more, 30 times or more, 40 times or more, 50 times or more of the total volume of the mesopores included in the mesoporous silica shell. For example, the volume of the hollow portion may be 100 times or less of the total volume of the mesopores included in the mesoporous silica shell. In this case, the active substance can be easily supported within the hollow portion, and the supported active substance can remain stable without being released.

In some embodiments, the silicon-carbon molar ratio of the silica shell may be 1:0.2 to 1:1. Preferably, the silicon-carbon molar ratio of the silica shell may be 1:0.3 to 1:1, 1:0.4 to 1:1, 1:0.5 to 1:1, 1:0.6 to 1:1, 1:0.7 to 1:1, or 1:0.8 to 1:1. In this case, the size and total volume of the mesopores of the silica shell and the volume of the hollow portion can be effectively controlled within the ranges described above. Therefore, the active substance can be easily supported within the hollow portion, and the supported active substance can remain stable without being released.

The term "active substance" collectively refers to a substance that exhibits a specific effect on a living organism. The active substance may include cosmetics, pharmaceuticals, diagnostic agents, pesticides, catalysts, and the like. In some embodiments, the solvent itself may function as the active substance.

In some embodiments, the hollow mesoporous silica rod may comprise an active substance supported within the hollow portion. For example, the active substance may be introduced into the hollow mesoporous silica rod. The active substance may comprise a hydrophilic or hydrophobic substance.

In some embodiments, the active substance may comprise a hydrophobic substance. For example, the hydrophobic substance may be dissolved in a hydrophobic solvent.

In some embodiments, the active substance may include any one selected from mineral oil, oleic acid, methyl oleate, vegetable oil, caprylic/capric triglyceride, ascorbyl tetraisopalmitate, oil-soluble licorice extract, alpha-bisabolol, retinyl palmitate, tocopherol, madeca, or terpenes.

In some embodiments, madeca may include madecassoside, madecassic acid, asiaticoside, asiatic acid, and the like.

The vegetable oil may include soybean oil, sunflower oil, palm oil, coconut oil, argan oil, olive oil, avocado oil, canola oil, olive oil, and the like.

The terpenes may include terpenes or terpenoids. For example, the terpenes may include monoterpenes, monoterpenoids, sesquiterpenoids, diterpenoids, sesterterpenoids, triterpenoids, sesquiterpenoids, tetraterpenoids, polyterpenoids, norisoprenoids, hemiterpenoids, and the like. The terpenes may have linear (acyclic) or cyclic structures. For example, the cyclic structures may include monocyclic, bicyclic, tricyclic, tetracyclic, and the like.

The terpenes may include squalene, vitamin A, ocimene, limonene, linalool, eucalyptol, geraniol, citronellol, and the like.

The active substance may be a hydrophobic substance itself, or a hydrophobic substance containing a functional substance dissolved therein, such as a substance for whitening, ameliorating wrinkles, removing body hair, alleviating acne-prone skin, alleviating hair loss symptoms, enhancing elasticity, or moisturizing the skin, ameliorating skin-related diseases, regulating skin physiological activity, or regulating skin immune function.

When a hollow mesoporous silica rod is dispersed in a hydrophobic solvent in which an active substance has been dissolved, the active substance may be supported within the hollow mesoporous silica rod through the mesopores, along with the hydrophobic solvent. When a hydrophilic solvent such as water is added in excess to a hydrophobic solvent, an oil-in-water emulsion may be formed. Since the surface of the silica shell is hydrophilic and is dispersed better in water than in the hydrophobic solvent, the dispersion medium may be changed from the hydrophobic solvent to water. In this case, since the hydrophobic solvent and the active substance supported within the hollow portion do not mix with water, they may be stably maintained in the state of being supported within the hollow portion without leaking out to the outside.

In some embodiments, the hydrophobic solvent may include an unsaturated hydrocarbon solvent. The unsaturated hydrocarbon solvent may include an at least partially unsaturated hydrocarbon having 10 to 50 carbon atoms. Preferably, it may have 10 to 40, 20 to 50, or 20 to 40 carbon atoms. The unsaturated hydrocarbon may comprise 1 to 20, 1 to 10, 1 to 5, 5 to 20, or 5 to 10 unsaturated bonds (double bonds or triple bonds). For example, the unsaturated hydrocarbon solvent may include squalene.

In some embodiments, the hollow mesoporous silica rod may comprise a capping layer that caps the mesoporous pores of the silica shell. The capping layer may block the entry of a substance through the mesoporous pores. In some embodiments, the capping layer may fill the internal spaces of the mesoporous pores of the silica shell or partially or completely cover the outer surface of the silica shell.

For example, the active substance may include a hydrophilic substance. The hydrophilic substance may be dissolved in a hydrophilic solvent. Since the surface of the silica shell is hydrophilic, the medicinal substance may be easily moved from the inside to the outside within the hydrophilic solvent, and during this process, the medicinal substance introduced into the hollow portion may also be easily released to the outside. In this case, the mesoporous pores may be capped with the capping layer to block the release of the medicinal substance within the hollow portion.

In some embodiments, the capping layer may be formed of inorganic silica. In this case, the outer surface of the silica shell may be easily capped through polymerization reaction of inorganic silica precursors, thereby effectively blocking the release of the internal medicinal substance.

In one embodiment of the present invention, there is provided a method for producing a hollow mesoporous silica rod. The method for producing a hollow mesoporous silica rod may comprise the steps of: preparing a rod-shaped inorganic silica core; forming a mesoporous organic silica shell on the surface of the inorganic silica core to produce a core-shell silica rod; and removing the inorganic silica core within the core-shell silica rod.

Referring to Figure 5, a hollow mesoporous silica rod can be produced by preparing a rod-shaped inorganic silica core; forming a mesoporous organic silica shell on the surface of the inorganic silica core to produce a core-shell silica rod; and removing the inorganic silica core within the core-shell silica rod.

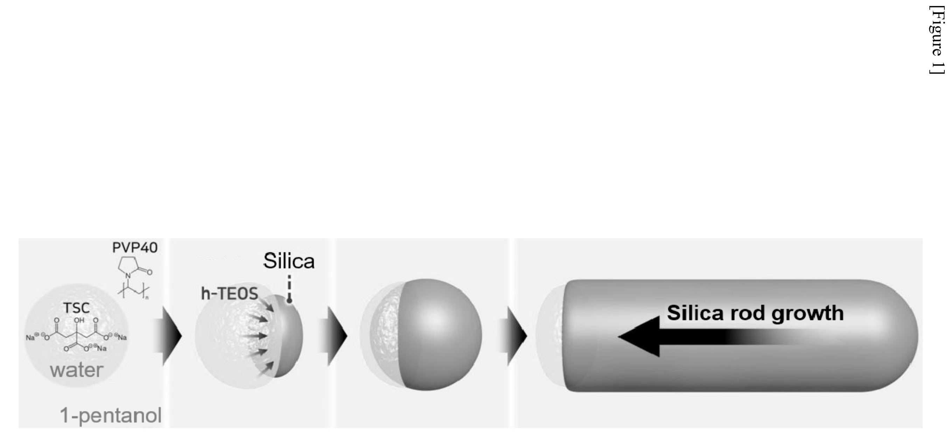

Referring to Figure 1, in the step of preparing a rod-shaped inorganic silica core, an oil-in-water emulsion comprising an alcoholic solvent and a polymeric surfactant can be prepared. The oil-in-water emulsion can be produced by mixing an alcoholic solvent and a surfactant with a hydrophilic solvent, such as water.

In some embodiments, the alcoholic solvent may include a monohydric or polyhydric alcohol having 2 to 20 carbon atoms. For example, the alcoholic solvent may include methanol, ethanol, propanol, butanol, pentanol, hexanol, heptanol, octanol, and the like.

The polymeric surfactant may include polyvinylpyrrolidone or a polyoxyalkylene block copolymer. In some embodiments, the polymeric surfactant may be at least one selected from the group consisting of polyvinylpyrrolidone (PVP) having an average molecular weight of 10,000 Da to 100,000 Da, Brij 52, Brij 58, Brij 30, Brij 76, Brij 78, Brij 97, Brij 35, Tritox X-100, Trton C-114, Tween 20, Tween 40, Tween 60, Tween 80, Span 40, Pluronic L121, Pluronic L64, Pluronic P103, Pluronic P123, Pluronic F68, Pluronic F127, Pluronic F88, Tetronic 908, Tetronic 901, and Tetronic 90R4.

In some embodiments, an ionic stabilizer may be added to the oil-in-water emulsion. The ionic stabilizer may include trisodium citrate, etc. In this case, it can stably maintain the emulsified state of the oil-in-water emulsion.

An inorganic silica precursor may be added to the oil-in-water emulsion to grow the inorganic silica in a rod shape.

The inorganic silica precursor may include a tetraalkoxysilane. For example, the tetraalkoxysilane may be a compound in which four alkoxy groups are substituted on the silicon central atom. The alkoxy groups may have 1 to 6 carbon atoms. For example, the alkoxy groups may be methoxy, ethoxy, propoxy, butoxy, pentoxy, or hexoxy. The four alkoxy groups of the tetraalkoxysilane may be the same or different. For example, the inorganic silica precursor may be tetramethyl orthosilicate, tetraethyl orthosilicate, or tetrapropyl orthosilicate.

The inorganic silica precursor is hydrophobic and can be preferentially dissolved in the alcoholic solvent, then diffused in water, and hydrated. The hydrated precursor can first form spherical inorganic silica through condensation reaction. Remaining water in the oil-in-water emulsion adheres to one side of the silica, and the hydrated precursor diffused in the water can grow the spherical inorganic silica only in one direction to form inorganic silica rods. In this case, the aspect ratio of the silica rods can be controlled by adjusting the amount of the inorganic silica precursor used.

In some embodiments, the inorganic silica may be formed by the Stöber process. For example, a silica-forming catalyst, such as ammonia, may be added to the oil-in-water emulsion, and in the presence of the catalyst, the inorganic silica precursor may form SiO₂. An alcohol corresponding to the alkoxy group of the tetraalkoxysilane may be additionally added to the oil-in-water emulsion.

In some embodiments, the inorganic silica rod formation reaction may be performed at a temperature of 20°C to 37°C. In some embodiments, the inorganic silica rod formation reaction may be performed for 12 to 18 hours.

In some embodiments, the step of preparing a rod-shaped inorganic silica core may comprise the steps of: preparing an inorganic silica nanoseed by adding a silica precursor to a solution comprising an alcoholic solvent and a silica-forming catalyst; preparing an oil-in-water emulsion comprising the inorganic silica nanoseed, an alcoholic solvent, and a surfactant; and adding an inorganic silica precursor to the oil-in-water emulsion to grow the inorganic silica in a rod shape.

In some embodiments, the silica-forming catalyst may include ammonia, an amine-based compound, an alkali metal or alkaline earth metal hydroxide, a transition metal catalyst, an inorganic acid such as hydrochloric acid, and the like.

For example, the step of preparing a rod-shaped inorganic silica core may further comprise the step of preparing an inorganic silica nanoseed having a size (diameter) of 200 nm to 1 µm. In this case, the inorganic silica nanoseed may be formed by the Stöber process. The inorganic silica nanoseed may be spherical.

In some embodiments, the inorganic silica nanoseed may be formed by adding an inorganic silica precursor to a solution comprising an alcoholic solvent and a silica-forming catalyst and stirring the solution. For example, the inorganic silica seed may be synthesized by adding an inorganic silica precursor to a solution comprising ethanol, water, and ammonia and stirring the solution.

The inorganic silica seed may be formed at a temperature of 20°C to 37°C. The inorganic silica seed may be formed by reaction for 12 to 18 hours.

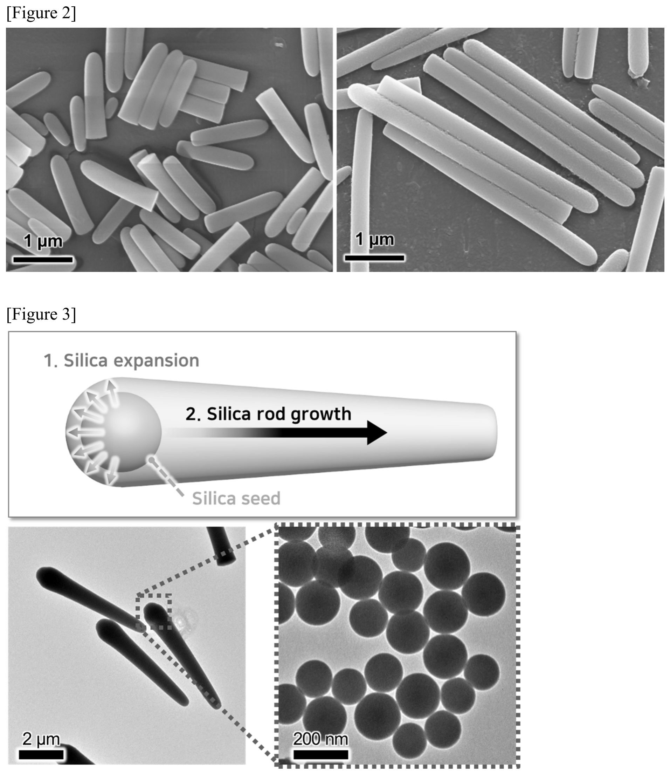

Referring to Figure 3, the inorganic silica seed may be added to the oil-in-water emulsion. In this case, a rod-shaped inorganic silica core may be synthesized by one-dimensionally growing from the inorganic silica seed, and a more uniform rod-shaped inorganic silica core may be produced by the inorganic silica seed.

The oil-in-water emulsion may comprise an alcoholic solvent and a surfactant.

The alcoholic solvent may include a monohydric or polyhydric alcohol having 2 to 20 carbon atoms. For example, the alcoholic solvent may include methanol, ethanol, propanol, butanol, pentanol, hexanol, heptanol, octanol, and the like. In some embodiments, the alcoholic solvent may be a mixed solvent of two or more alcohols. For example, the alcoholic solvent may include ethanol and pentanol.

The surfactant may include a polymeric surfactant. For example, the polymeric surfactant may include polyvinylpyrrolidone or a polyoxyalkylene block copolymer. In some embodiments, the polymeric surfactant may be at least one selected from the group consisting of polyvinylpyrrolidone (PVP) having an average molecular weight of 10,000 Da to 100,000 Da, Brij 52, Brij 58, Brij 30, Brij 76, Brij 78, Brij 97, Brij 35, Tritox X-100, Trton C-114, Tween 20, Tween 40, Tween 60, Tween 80, Span 40, Pluronic L121, Pluronic L64, Pluronic P103, Pluronic P123, Pluronic F68, Pluronic F127, Pluronic F88, Tetronic 908, Tetronic 901, and Tetronic 90R4.

In some embodiments, the oil-in-water emulsion may further comprise an ionic surfactant and/or a silica-forming catalyst.

The ionic surfactant may include a cationic surfactant, such as cetrimonium bromide (CTAB), cetrimonium chloride (CTAC), cetylpyridinium chloride (CPC), benzalkonium chloride (BAC), benzethonium chloride (BZT), dimethyldioctadecylammonium chloride, or dioctadecyldimethylammonium bromide (DODAB).

The silica-forming catalyst may include ammonia, an amine-based compound, an alkali metal or alkaline earth metal hydroxide, a transition metal catalyst, an inorganic acid such as hydrochloric acid, and the like.

An inorganic silica precursor may be added to the oil-in-water emulsion to grow the inorganic silica in a rod shape.

The inorganic silica precursor may include a tetraalkoxysilane. For example, the tetraalkoxysilane may be a compound in which four alkoxy groups are substituted on the silicon central atom. The alkoxy groups may have 1 to 6 carbon atoms. For example, the alkoxy groups may be methoxy, ethoxy, propoxy, butoxy, pentoxy, or hexoxy. The four alkoxy groups of the tetraalkoxysilane may be the same or different. For example, the inorganic silica precursor may be tetramethyl orthosilicate, tetraethyl orthosilicate, or tetrapropyl orthosilicate.

In some embodiments, the inorganic silica rod may be formed at a temperature of 20°C to 70°C, 20°C to 60°C, 20°C to 50°C, 20°C to 40°C, or 20°C to 37°C.

In some embodiments, the inorganic silica rod may be formed by reaction for 12 to 18 hours.

In some embodiments, the aspect ratio of the inorganic silica rod may be matched with the aspect ratio of the hollow mesoporous silica rod. For example, the aspect ratio may be 1:2 to 1:100, 1:2 to 1:90, 1:2 to 1:80, 1:2 to 1:70, 1:2 to 1:60, 1:2 to 1:50, 1:2 to 1:40, 1:2 to 1:30, 1:2 to 1:20, 1:3 to 1:100, 1:3 to 1:90, 1:3 to 1:80, 1:3 to 1:70, 1:3 to 1:60, 1:3 to 1:50, 1:3 to 1:40, 1:3 to 1:30, 1:3 to 1:20, 1:4 to 1:100, 1:4 to 1:90, 1:4 to 1:80, 1:4 to 1:70, 1:4 to 1:60, 1:4 to 1:50, 1:4 to 1:40, 1:4 to 1:30, 1:4 to 1:20, 1:5 to 1:100, 1:5 to 1:90, 1:5 to 1:80, 1:5 to 1:70, 1:5 to 1:60, 1:5 to 1:50, 1:5 to 1:40, 1:5 to 1:30, or 1:5 to 1:20.

The length of the inorganic silica rod (the length of the major axis) may be 10 µm or greater. For example, the length of the inorganic silica rod may be 12 µm or greater, 15 µm or greater, or 18 µm or greater. In addition, the length of the inorganic silica rod may be 100 µm or less.

The thickness of the inorganic silica rod (the length of the first minor axis or the second minor axis or the diameter) may be 5 µm or less. For example, the thickness of the inorganic silica rod may be 4 µm or less, 3 µm or less, or 2 µm or less. In addition, the thickness of the inorganic silica rod may be 0.1 µm or greater.

A core-shell silica rod may be produced by forming a mesoporous organic silica shell on the surface of the inorganic silica core. For example, the mesoporous organic silica shell may cover part or all of the surface of the inorganic silica core.

In some embodiments, the mesoporous organic silica shell may be formed by reacting the inorganic silica core with a surfactant and an organic silica precursor in a mixed solvent of water and an alcoholic solvent. For example, the inorganic silica core may be dispersed in the mixed solvent, and the surfactant and the organic silica precursor may be added and reacted to form a mesoporous organic silica shell. The surfactant may induce the mesopores of the organic silica shell.

In some embodiments, the alcoholic solvent may include a monohydric or polyhydric alcohol having 2 to 20 carbon atoms. For example, the alcoholic solvent may include methanol, ethanol, propanol, butanol, pentanol, hexanol, heptanol, octanol, and the like.

For example, the ratio of water and the alcoholic solvent in the mixed solvent may be 40:60 to 90:10, 40:60 to 80:20, 40:60 to 70:30, 40:60 to 65:35, 50:50 to 90:10, 50:50 to 80:20, 50:50 to 70:30, 50:50 to 65:35, 60:40 to 90:10, 60:40 to 80:20, 60:40 to 70:20, or 60:40 to 65:35. In this case, a mesoporous organic silica shell may be effectively formed with a uniform thickness on the rod-shaped inorganic silica rod.

The surfactant may include an ionic surfactant. The surfactant may be dissolved in the alcoholic solvent and used.

In some embodiments, the ionic surfactant may include a cationic surfactant, such as cetrimonium bromide (CTAB), cetrimonium chloride (CTAC), cetylpyridinium chloride (CPC), benzalkonium chloride (BAC), benzethonium chloride (BZT), dimethyldioctadecylammonium chloride, or dioctadecyldimethylammonium bromide (DODAB).

In some embodiments, a silica-forming catalyst may be used in the organic silica shell formation reaction. The silica-forming catalyst may include ammonia, an amine-based compound, an alkali metal or alkaline earth metal hydroxide, a transition metal catalyst, an inorganic acid such as hydrochloric acid, and the like.

The organic silica precursor includes an organic alkoxysilane. The organic alkoxysilane is a compound in which the silicon central atom is substituted with a hydrocarbon group in addition to alkoxy groups, and may include an alkoxysilylalkylene in which two or more alkoxysilyl groups are linked by an alkylene group.

In some embodiments, the organic silica precursor may be any one or more of BTSE (bis(triethoxysilyl)ethane), BTSEY (bis(triethoxysilyl)ethylene), BTES (bis-[3-(triethoxysilyl)tetrasulfide]), bis[3-(triethoxysilyl)propyl]tetrasulfide, 1,4-bis(triethoxysilyl)benzene, BTEB (bis(triethoxysilyl)phenylene), and BTEBP (bis(triethoxysilyl)-biphenyl).

In some embodiments, the organic silica shell may be formed through two or more shell formation steps. For example, the organic silica shell may be formed through a first shell formation step and a second shell formation step, wherein the first shell formation step is the same as described above. After the first shell formation step, the supernatant may be separated from the reaction solution, and the second shell formation step may be performed in the supernatant.

For example, the second shell formation step may use a smaller amount of ionic surfactant compared to the first shell formation step. For example, the ionic surfactant may be used in a lesser amount, such as about 70% or less, about 60% or less, or about 50% or less, in the second shell formation step compared to the first shell formation step.

In some embodiments, a silica-forming catalyst may not be used in the second shell formation step. For example, a silica-forming catalyst may be used in the first shell formation step, and a silica-forming catalyst may not be used in the second shell formation step.

The inorganic silica core within the core-shell silica rod may be removed. The internal space of the core-shell silica rod from which the inorganic silica core is removed may be provided as the hollow portion.

In some embodiments, the step of removing the inorganic silica core may comprise etching the core-shell silica rod with an alkaline solution. For example, the core-shell silica rod may be dispersed in water, and the alkaline solution may be added to the dispersion to remove the inorganic silica core. The alkaline solution has high etching selectivity for the inorganic silica core compared to the organic silica shell, and the inorganic silica core may be removed while the organic silica shell is preserved.

In some embodiments, the alkaline solution may comprise an alkali metal hydroxide. The alkali metal hydroxide may include LiOH, NaOH, KOH, and the like. For example, the alkaline solution may be an aqueous solution of the alkali metal hydroxide.

In some embodiments, the reaction temperature of the dispersion and the alkaline solution may be 50°C to 90°C. Preferably, the temperature of the alkaline solution may be 50°C to 80°C, 60°C to 90°C, or 60°C to 80°C. In this case, the alkaline solution may have high etching selectivity for the inorganic silica core compared to the organic silica shell.

In one embodiment of the present invention, there is provided a method for supporting an active substance within a hollow mesoporous silica rod. The method for supporting an active substance within a hollow mesoporous silica rod may comprise the steps of: mixing the hollow mesoporous silica rod with an active substance; and injecting the active substance into the hollow portion of the hollow mesoporous silica rod.

Referring to Figure 6, a hollow mesoporous silica rod may be dispersed in a solution comprising an active substance, an oil-in-water emulsion may be formed from the solution, and the solution may be moved into the hollow mesoporous silica rod, thereby supporting the active substance within the hollow mesoporous silica rod.

The active substance may include a hydrophobic substance or a hydrophilic substance. The active substance may be dissolved in an appropriate solvent. For example, the solution may comprise the solvent and the active substance. The solvent, solution, and active substance may each be hydrophilic or hydrophobic.

In some embodiments, the active substance comprises a hydrophobic substance, and the step of injecting the active substance may comprise forming an oil-in-water emulsion from a mixed solution of the hollow mesoporous silica rod and the active substance. As an oil-in-water emulsion is formed, a hydrophobic substance outside the hollow mesoporous silica rod may be injected into the hollow portion.

When a hollow mesoporous silica rod is dispersed in a hydrophobic solvent in which an active substance has been dissolved, the active substance may be supported within the hollow mesoporous silica rod through the mesopores, along with the hydrophobic solvent. When a hydrophilic solvent such as water is added in excess to a hydrophobic solvent, an oil-in-water emulsion may be formed. Since the surface of the organic silica shell is hydrophilic and is dispersed better in water than in the hydrophobic solvent, the dispersion medium may be changed from the hydrophobic solvent to water. In this case, since the hydrophobic solvent and the active substance supported within the hollow portion do not mix with water, they may be stably maintained in the state of being supported within the hollow portion without leaking out to the outside.

In some embodiments, the hydrophobic solvent may include an unsaturated hydrocarbon solvent. The unsaturated hydrocarbon solvent may include an at least partially unsaturated hydrocarbon having 10 to 50 carbon atoms. Preferably, it may have 10 to 40, 20 to 50, or 20 to 40 carbon atoms. The unsaturated hydrocarbon may comprise 1 to 20, 1 to 10, 1 to 5, 5 to 20, or 5 to 10 unsaturated bonds (double bonds or triple bonds). For example, the unsaturated hydrocarbon solvent may include squalene.

In some embodiments, the method for supporting an active substance may further comprise the step of removing any remaining active substance not injected into the hollow portion by centrifugation.

In some embodiments, the method for supporting an active substance may further comprise the step of capping mesoporous pores present in the shell of the silica rod into which the active substance has been injected.

For example, after the solution has been moved, the mesoporous pores of the organic silica shell may be capped. For example, the mesoporous pores may be filled or the mesoporous pores may be capped by forming a capping layer that covers the silica shell.

In some embodiments, the solution may comprise a hydrophilic solvent and a hydrophilic active substance. The hydrophilic active substance may be dissolved in the hydrophilic solvent. Since the surface of the organic silica shell is hydrophilic, the medicinal substance may be easily moved from the inside to the outside within the hydrophilic solvent, and during this process, the medicinal substance introduced into the hollow portion may also be easily released to the outside. In this case, the mesoporous pores may be capped with the capping layer to block the release of the medicinal substance within the hollow portion.

In some embodiments, the capping layer may be formed of inorganic silica. In this case, the outer surface of the organic silica shell may be easily capped through polymerization reaction of inorganic silica precursors, thereby effectively blocking the release of the internal medicinal substance. The capping layer may be formed using substantially the same method for the inorganic silica core.

In one embodiment of the present invention, there is provided a cosmetic composition comprising the hollow mesoporous silica rod as an active ingredient.

In some embodiments, the cosmetic composition may be used for whitening, ameliorating wrinkles, removing body hair, alleviating acne-prone skin, alleviating hair loss symptoms, enhancing elasticity, or moisturizing the skin.

The hollow mesoporous silica rod may be included in the cosmetic composition in an amount of 0.1 wt% to 10 wt%. Specifically, the hollow mesoporous silica rod may be included in an amount of 0.1 wt% to 9 wt%, 0.1 wt% to 8 wt%, 0.1 wt% to 6 wt%, 0.1 wt% to 5 wt%, 0.1 wt% to 3 wt%, 0.1 wt% to 2 wt%, 0.1 wt% to 1 wt%, 0.2 wt% to 10 wt%, 0.2 wt% to 9 wt%, 0.2 wt% to 8 wt%, 0.2 wt% to 6 wt%, 0.2 wt% to 5 wt%, 0.2 wt% to 3 wt%, 0.2 wt% to 2 wt%, 0.2 wt% to 1 wt%, 0.3 wt% to 10 wt%, 0.3 wt% to 9 wt%, 0.3 wt% to 8 wt%, 0.3 wt% to 6 wt%, 0.3 wt% to 5 wt%, 0.3 wt% to 3 wt%, 0.3 wt% to 2 wt%, 0.3 wt% to 1 wt%, 0.5 wt% to 10 wt%, 0.5 wt% to 9 wt%, 0.5 wt% to 8 wt%, 0.5 wt% to 6 wt%, 0.5 wt% to 5 wt%, 0.5 wt% to 3 wt%, 0.5 wt% to 2 wt%, or 0.5 wt% to 1 wt%.

For example, the content of the hollow mesoporous silica rod is not particularly limited, but may preferably be 0.1 to 5 parts by weight based on 100 parts by weight of the cosmetic composition.

The cosmetic composition may be in a formulation commonly used in cosmetics. In one embodiment, the composition may be produced in a formulation including a toner (skin lotion), skin softener, skin toner, astringent, lotion, milk lotion, moisturizing lotion, nourishing lotion, massage cream, nourishing cream, moisturizing cream, eye cream, hand cream, foundation, essence, nourishing essence, eye essence, pack, soap, cleansing foam, cleansing lotion, cleansing cream, body lotion, body cream, body cleanser, suspension, gel, powder, paste, mask pack, sheet, or aerosol composition. In addition, the composition may be applied in various forms suitable for providing moisture to the skin, such as a gel or cream, paste, or solid form, and specifically, may be a gel-cream form with a particle texture, such as sherbet or slush. Compositions of such formulations may be produced using methods conventional in the art.

In one embodiment, the composition may further comprise at least one selected from the group consisting of oils, purified water, emulsifying agents, dispersants, pigments, fragrances, sunscreens, sweeteners, vitamins, and metal ion sequestrants. In addition, in one embodiment, the composition may further comprise a preservative in an amount of 0.1 wt% to 3.0 wt%. The blending amounts of additional ingredients, such as oils, can be easily selected by those of ordinary skill in the art within a range that does not compromise the purpose and effects of the present invention.

In one embodiment of the present invention, there is provided a pharmaceutical composition comprising the hollow mesoporous silica rod as an active ingredient. For example, the hollow mesoporous silica rod may support an active substance therein, and the active substance can exhibit a pharmaceutically effective effect.

The hollow mesoporous silica rod may be included in the pharmaceutical composition in an amount of 0.1 wt% to 10 wt%. Specifically, the hollow mesoporous silica rod may be included in an amount of 0.1 wt% to 9 wt%, 0.1 wt% to 8 wt%, 0.1 wt% to 6 wt%, 0.1 wt% to 5 wt%, 0.1 wt% to 3 wt%, 0.1 wt% to 2 wt%, 0.1 wt% to 1 wt%, 0.2 wt% to 10 wt%, 0.2 wt% to 9 wt%, 0.2 wt% to 8 wt%, 0.2 wt% to 6 wt%, 0.2 wt% to 5 wt%, 0.2 wt% to 3 wt%, 0.2 wt% to 2 wt%, 0.2 wt% to 1 wt%, 0.3 wt% to 10 wt%, 0.3 wt% to 9 wt%, 0.3 wt% to 8 wt%, 0.3 wt% to 6 wt%, 0.3 wt% to 5 wt%, 0.3 wt% to 3 wt%, 0.3 wt% to 2 wt%, 0.3 wt% to 1 wt%, 0.5 wt% to 10 wt%, 0.5 wt% to 9 wt%, 0.5 wt% to 8 wt%, 0.5 wt% to 6 wt%, 0.5 wt% to 5 wt%, 0.5 wt% to 3 wt%, 0.5 wt% to 2 wt%, or 0.5 wt% to 1 wt%.

In one embodiment, the pharmaceutical composition may comprise conventional pharmaceutically acceptable carriers, excipients, or additives. The pharmaceutical composition may be formulated using conventional methods, and may be produced in various oral dosage forms, such as tablets, pills, powders, capsules, syrups, emulsions, and microemulsions, or parenteral dosage forms, such as intramuscular, intravenous, or subcutaneous administration.

When the pharmaceutical composition is produced in the form of an oral dosage form, examples of additives or carriers used may include cellulose, calcium silicate, corn starch, lactose, sucrose, dextrose, calcium phosphate, stearic acid, magnesium stearate, calcium stearate, gelatin, talc, surfactants, suspending agents, emulsifying agents, diluents, and the like. When the pharmaceutical composition of the present invention is produced in the form of an injection, additives or carriers may include water, saline, glucose aqueous solution, pseudo-sugar aqueous solution, alcohol, glycol, ether (e.g., polyethylene glycol 400), oil, fatty acid, fatty acid ester, glyceride, surfactants, suspending agents, emulsifying agents, and the like.

Hereinafter, the present invention will be described in more detail by way of the following examples. However, the following examples are only for illustrating the present invention, and the scope of the present invention is not limited only to these examples.

### Example 1.1: Production of inorganic silica rods

A PVP/PeOH solution was prepared by dissolving polyvinylpyrrolidone (PVP, Aldrich) with an average molecular weight of 40,000 in 1-pentanol (PeOH) at a concentration of 1 g/10 mL.

An emulsion was produced by adding 0.96 mL of a 6.75 mM trisodium citrate (TSC) aqueous solution, 3 mL of ethanol, and 0.7 mL of ammonia to 30 mL of PVP/PeOH solution and vigorously mixing for 1 minute using a stirrer.

Tetraethyl orthosilicate (TEOS), a silica precursor, was added to the emulsion in an amount of 0.1 mL to 5 mL depending on the target length, and the mixture was allowed to react in a constant-temperature water bath at 20°C to 37°C for 18 hours to produce silica rods.

The produced silica rod was washed with ethanol, acetic acid/ethanol (10 v/v%), and water by centrifugation (5,000 rpm, 4 minutes), and additional centrifugation (1800 rpm, 7 minutes) with ethanol was performed to remove byproducts, and then dried to obtain purified inorganic silica rods.

Figure 2 is a scanning electron microscope (SEM) image of inorganic silica rods produced according to the example. Referring to Figure 2, it is confirmed that inorganic silica rods with various aspect ratios were produced using a simple method.

### Example 1.2: Production of inorganic silica rods using silica seeds

A mixed solution of 30 mL of ethanol, 1.5 mL of water, and 4.0 mL of ammonia was stirred at 400 rpm at 20°C to 37°C, 4 mL of TEOS, a silica precursor, was added, and then the mixture was allowed to react for 18 hours to obtain inorganic silica nanoseeds (300 to 450 nm).

The synthesized inorganic silica nanoseeds were washed several times with ethanol and water by centrifugation (5,000 rpm, 4 minutes) and dispersed in 60 mL of water.

A PVP/PeOH/EtOH solution was prepared by dissolving polyvinylpyrrolidone (PVP, Aldrich) with an average molecular weight of 55,000 in 1-pentanol (PeOH) at a concentration of 1 g/10 mL and then adding 80 mL of ethanol (EtOH).

An emulsion was produced by adding 0.8 mL of the synthesized inorganic silica nanoseed dispersion, 1.12 mL of 7.5 mM trisodium citrate (TSC) aqueous solution, and 1.12 mL of ammonia to 40 mL of PVP/PeOH/EtOH solution and vigorously mixing for 1 minute using a stirrer.

0.1 mL to 5 mL of tetraethyl orthosilicate (TEOS), a precursor, was added to the emulsion, and the mixture was allowed to react in a constant-temperature water bath at 20°C to 37°C for 12 to 18 hours to produce silica rods.

The produced silica rod was washed with ethanol, acetic acid/ethanol (10 v/v%), and water by centrifugation (5,000 rpm, 4 minutes), and additional centrifugation (1800 rpm, 7 minutes) with ethanol was performed to remove byproducts, and then dried to obtain purified inorganic silica rods.

Figure 3 is a transmission electron microscope (TEM) image of inorganic silica rods produced according to some embodiments, and Figure 4 is an optical microscope and scanning electron microscope (SEM) image of inorganic silica rods produced according to some embodiments. Referring to Figures 3 and 4, it was confirmed that inorganic silica rods were produced using inorganic silica nanoparticles as seeds.

### Example 2: Production of hollow mesoporous silica rods

The hollow mesoporous silica rods (HSMR) were produced from the silica rods according to the process shown in Figure 5.

The inorganic silica rods (28.5 mg) produced in Example 1.1 were dispersed in 40 mL of a mixed solvent of water and methanol (40:60 to 90:10, v/v).

1 mL of cetrimonium bromide (CTAB) solution (1 g/10 mL in methanol), 50 µL of 1,2-bis(triethoxysilyl)ethane (BTSE), and 400 µL of ammonia were added to the reaction solution, and the mixture was allowed to react on a rotator (3 rpm) for 2 hours.

The supernatant was transferred to a new vessel, and then 500 µL of CTAB solution (1 g/10 mL in methanol) and 50 µL of BTSE were added, and the mixture was allowed to react on a rotator (3 rpm) for 2 hours.

The supernatant was collected, washed with acetic acid/ethanol (10 v/v%), ethanol, and water by centrifugation (5000 rpm, 4 minutes), and dispersed in 40 mL of water.

The dispersed solution was heated to 70°C, 2 mL of NaOH (0.5 M) was added, and the mixture was allowed to react for 20 minutes to etch the internal inorganic silica rods. The mixture was washed with acetic acid/ethanol (10 v/v%), water, and ethanol by centrifugation (5000 rpm, 4 minutes), and dried to produce hollow mesoporous silica rods.

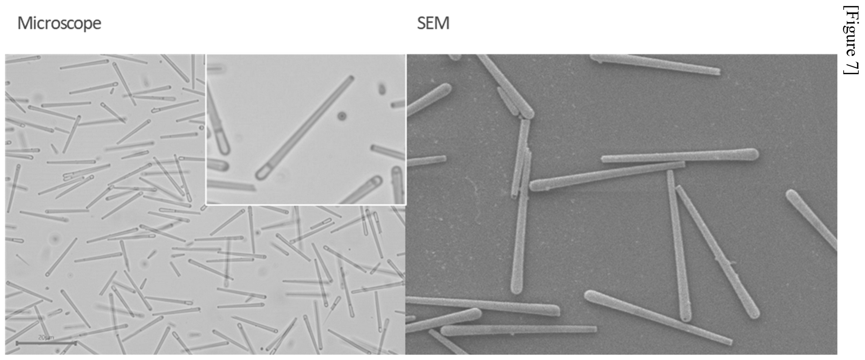

Figures 6 and 7 are transmission electron microscope (TEM), optical microscope and scanning electron microscope (SEM) images of hollow mesoporous silica rods produced based on the inorganic silica rods shown in Figure 2. Referring to Figures 6 and 7, it is confirmed that hollow mesoporous silica rods with various aspect ratios and shapes were produced using a simple method.

### Example 3: Supporting a hydrophobic substance

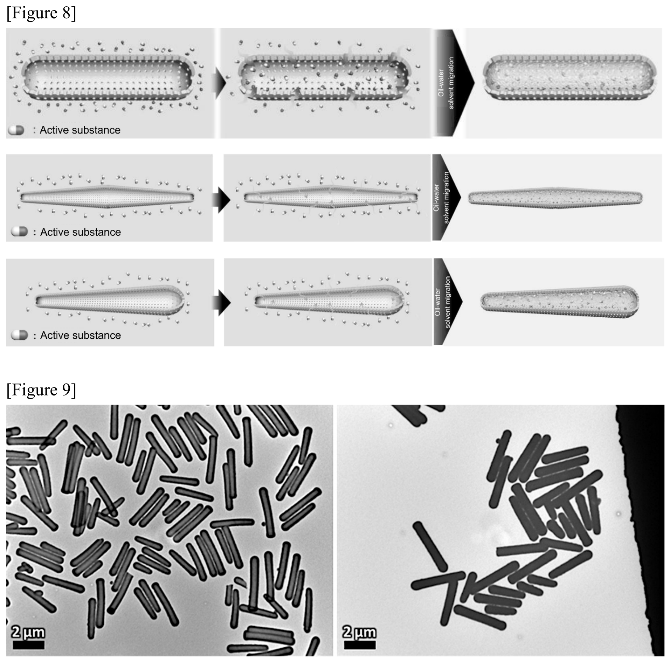

Referring to Figure 8, an active substance was supported within hollow mesoporous silica rods.

9 mg of hollow mesoporous silica rods were dispersed in 1 mL of squalene (Sigma Aldrich). 4 mL of water was added to the dispersion to form an oil-in-water (O/W) emulsion. The silica rods were filtered and dried to remove squalene and water, thereby producing hollow mesoporous silica rods loaded in which squalene has been supported.

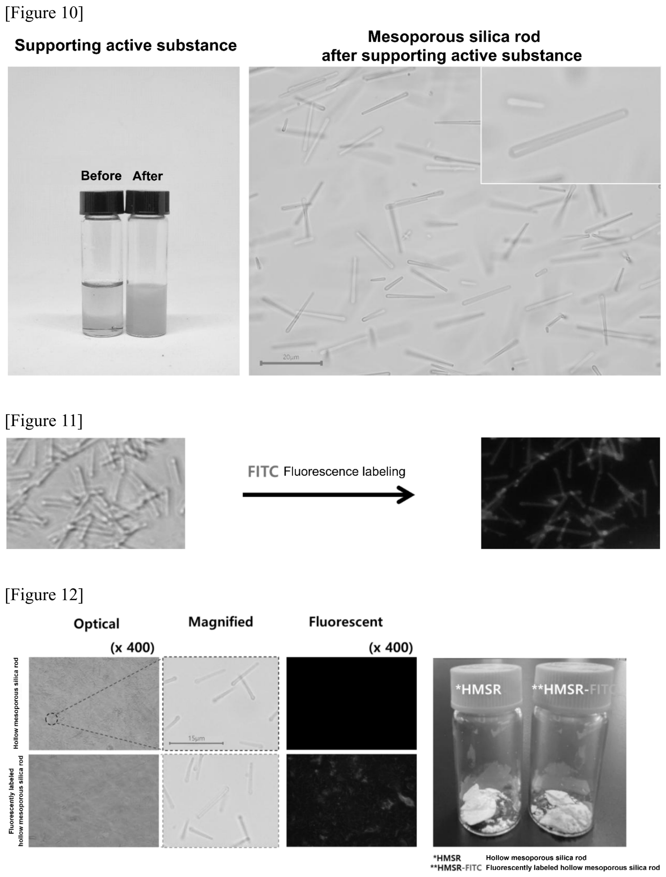

Figure 9 is a microscope image of mesoporous silica rods before and after supporting squalene, and Figure 10 is a photograph and an optical microscope image showing the observed changes before and after supporting madeca. Referring to Figures 9 and 10, it was confirmed that squalene and madeca were stably supported into the inner hollow portion of the hollow mesoporous silica rods.

### Example 4.1: Fluorescent labeling (tagging) of hollow mesoporous silica rods

The hollow mesoporous silica rods and the active substances were fluorescently labeled with green or red, respectively, in order to facilitate the evaluation of the incorporation of the active substances into the hollow mesoporous silica rods and *ex vivo* skin permeation.

18 mg of the green fluorescent substance FITC (fluorescein isothiocyanate) was weighed and placed in a reaction vessel, and then 3 mL of ethanol (purity 95% to 100%) was added. Thereafter, the mixture was stirred at room temperature for 10 to 30 minutes using a stirrer, and then 20 µL of APTMS ((3-aminopropyl)triethoxysilane) was added and stirred at room temperature for 12 to 18 hours to obtain a green fluorescence labeling solution. Reaction scheme 1 below is a schematic diagram of the chemical reaction for the green fluorescence labeling solution.

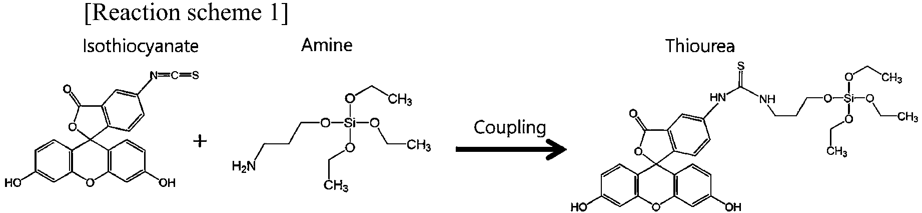

The green fluorescence labeling solution was added to the reaction solution of Example 2 so that the weight ratio of inorganic silica rods to green fluorescence labeling solution was 10:1. The mixture was stirred at room temperature for 18 hours and then centrifuged at 2,000 rpm for 4 minutes. The supernatant was removed from the centrifuged product, washed with 3 mL of ethanol, and then dried to produce green fluorescently labeled hollow mesoporous silica rods.

Figure 11 is a fluorescence microscope image of hollow mesoporous silica rods fluorescently labeled with FITC. Referring to Figure 11, it was directly confirmed that the hollow mesoporous silica rods were fluorescently labeled with green fluorescence.

Figure 12 is an optical microscope image and a fluorescence microscope image and a photograph of hollow mesoporous silica rods and fluorescently labeled hollow mesoporous silica rods. Referring to Figure 12, the difference in fluorescence labeling was confirmed by visual observation and microscopic observation before and after fluorescence labeling.

### Example 4.2: Fluorescence labeling of active substance

The hydrophobic active substance was vitamin B1 (thiamine), and the fluorescent substance for labeling was Cy5, a representative red fluorescent substance. For chemical reaction, one end of Cy5 was in the NHS-ester form. Reaction scheme 2 below is a schematic diagram of chemical reaction for red fluorescence labeling of the active substance.

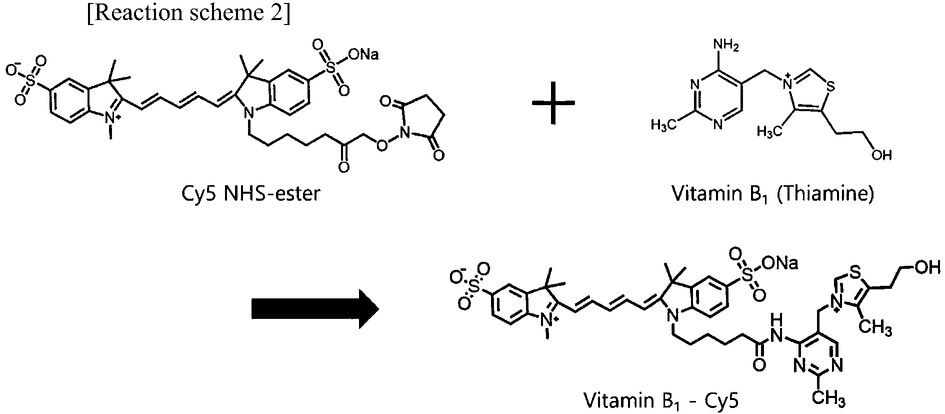

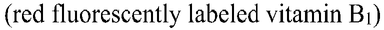

100 mg of thiamine (Sigma Aldrich) was weighed and placed in a reaction vessel, and 10 mL of ethanol was added. Thereafter, the mixture was stirred for 30 minutes to 1 hour using a stirrer, and then 2 mg of Cy5 NHS-ester was added, and the mixture was stirred at room temperature for 12 to 18 hours. The reaction residue was removed using a 500 Da CE Membrane and then dried to obtain fluorescently labeled vitamin B1 (thiamine).

### Example 5: Evaluation of the supported state of active substance in hollow mesoporous silica rods

The fluorescently labeled hydrophobic active substance (vitamin B1) of Example 4.2 was supported within the fluorescently labeled hollow mesoporous silica rods of Example 4.1, and the supported state was confirmed using a fluorescence microscope.

5 mg of the fluorescently labeled active substance was added to 1 mL of mineral oil (Sigma Aldrich) or oleic acid (Sigma Aldrich) and stirred until sufficiently dissolved. 9 mg of the fluorescently labeled hollow mesoporous silica rods were added and then dispersed. 4 mL of water was added to the dispersion to form an oil-in-water (O/W) emulsion. The silica rods were filtered and dried to remove mineral oil, oleic acid, and water, thereby producing green fluorescently labeled hollow mesoporous silica rods in which a red fluorescently labeled active substance has been supported.

1 mg of the fluorescently labeled active substance-supported hollow mesoporous silica rods was dispersed in 1 mL of water, and then fluorescence microscope images were obtained.

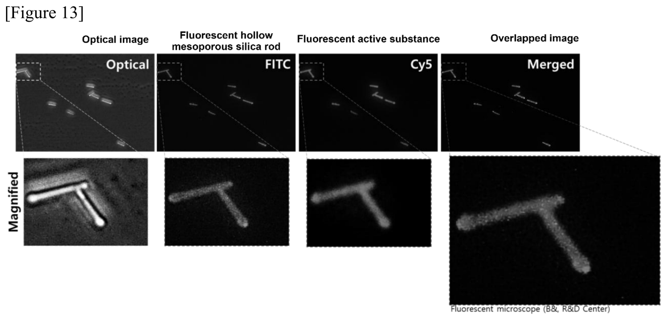

Figure 13 is a fluorescence microscope image of the fluorescently labeled active substance-supported hollow mesoporous silica rods. By overlapping the fluorescence images of the green fluorescence channel and the red fluorescence channel, it was confirmed that the active substance was well supported within the hollow mesoporous silica rods.

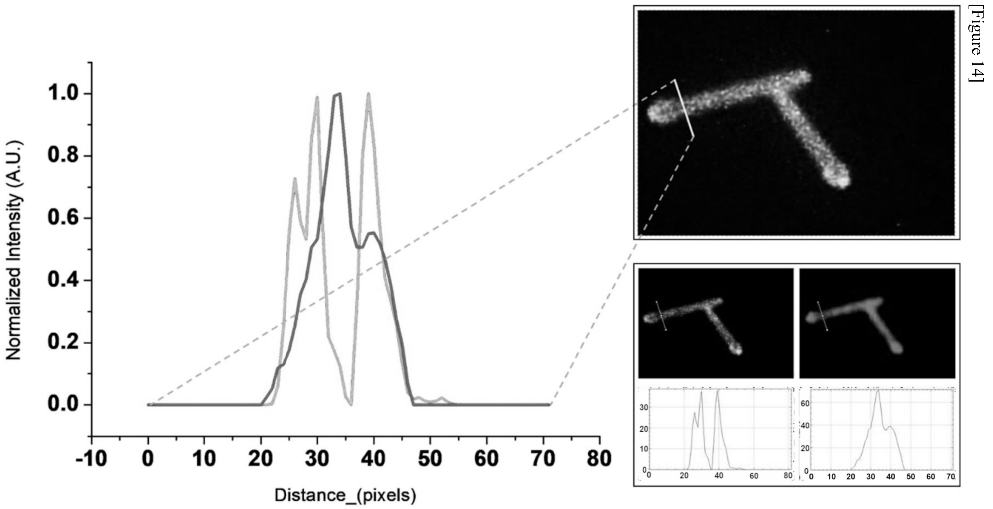

Figure 14 is a graph of red and green fluorescence intensities for the same cross-section of the fluorescently labeled active substance-supported hollow mesoporous silica rods. Referring to the graph in Figure 14, it was confirmed that the shell portion was surrounded by the hollow mesoporous silica rods, and the interior was filled with the active substance.

### Example 6: Confirmation of skin permeation and active substance delivery

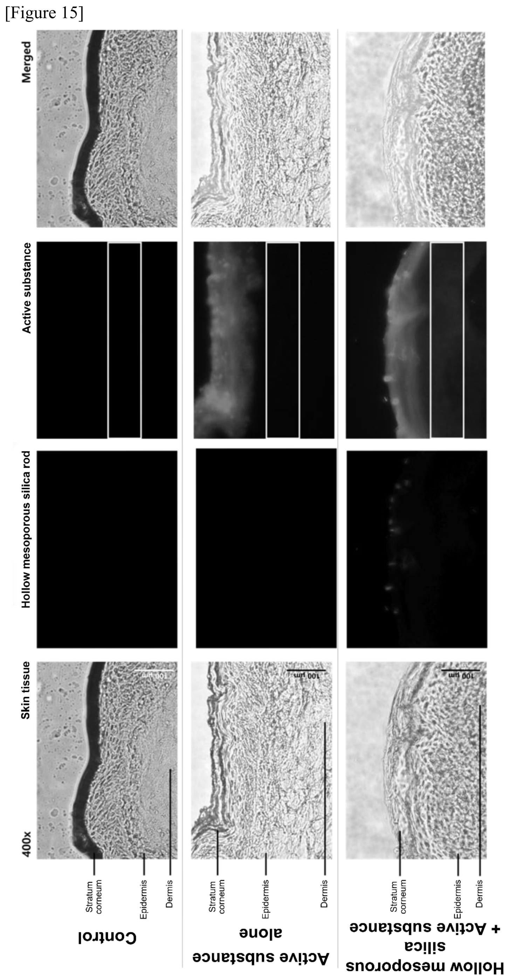

20 µL (10 mg/mL concentration) of water dispersion of the fluorescently labeled active substance-supported hollow mesoporous silica rods of Example 5 was applied to research pig skin (whole skin, 2 cm x 2 cm Franz cell membrane) for 5 minutes, and then washed to produce a cryoblock and obtain a tissue cross-section slide. The tissue cross-section slide was photographed under a fluorescence microscope to obtain Figure 15.

Figure 15 is an optical microscope image and a fluorescence microscope image comparing the degree of penetration of the active substance in a group treated with an example sample, a group treated with an active substance alone, and a control group in the research pig skin (Franz cell membrane).

Referring to Figure 15, in the control group and the group treated with active substance alone, no red fluorescence signal was detected within the epidermis, excluding the stratum corneum. However, in the group treated with the hollow mesoporous silica rods in which the active substance has been supported, it was confirmed that the active substance was delivered to the inside of the epidermis.

In particular, in the group treated with an example sample, it was confirmed that the fluorescence signal of the hollow mesoporous silica appeared only in the stratum corneum, whereas the active substance was delivered through the stratum corneum into the inside of the epidermis.

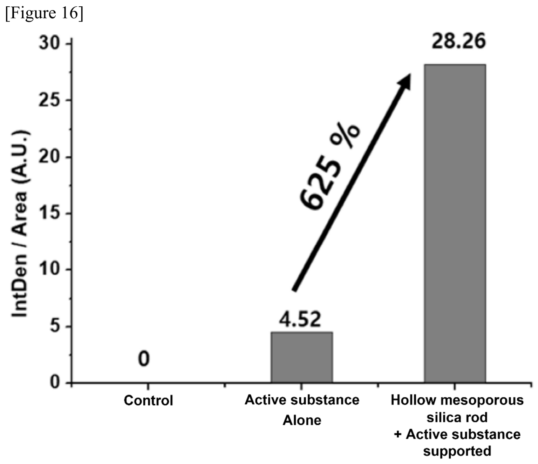

Figure 16 is a graph showing the fluorescence intensity within the epidermis, excluding the stratum corneum, of the fluorescent image of the active substance in Figure 15. When the total fluorescence intensity of the inside of the epidermis for each sample was measured and divided by the measured area to calculate the fluorescence intensity per unit area and compared, the experimental group treated with the active substance supported in hollow mesoporous silica rods showed a skin penetration amount 625% higher than the group treated with the active substance alone, which objectively confirmed that the degree of penetration of the active substance to the inside of the epidermis was excellent.

## Claims

1. A hollow mesoporous silica rod comprising:
a mesoporous silica shell; and
a hollow portion within the silica shell,
wherein the silica shell is rod-shaped and has an aspect ratio of 1:2 to 1:100.

2. The hollow mesoporous silica rod according to claim 1, wherein the silica shell has a thickness of 5 nm to 100 nm.

3. The hollow mesoporous silica rod according to claim 1, wherein the volume of the hollow portion is 50% to 99% based on the volume defined by the outer surface of the silica shell.

4. The hollow mesoporous silica rod according to claim 1, wherein the volume of the hollow portion is 10 times or more of the total volume of the mesopores included in the mesoporous silica shell.

5. The hollow mesoporous silica rod according to claim 1, wherein the silica shell is formed of organic silica.

6. The hollow mesoporous silica rod according to claim 1, wherein the hollow mesoporous silica rod further comprises an active substance in the hollow portion.

7. The hollow mesoporous silica rod according to claim 6, wherein the active substance is a hydrophobic substance.

8. The hollow mesoporous silica rod according to claim 7, wherein the active substance includes any one selected from mineral oil, oleic acid, methyl oleate, vegetable oil, caprylic/capric triglyceride, ascorbyl tetraisopalmitate, oil-soluble licorice extract, alpha-bisabolol, retinyl palmitate, tocopherol, madeca, or terpenes.

9. The hollow mesoporous silica rod according to claim 1, wherein the hollow mesoporous silica rod further comprises a capping layer that caps the mesoporous pores of the silica shell.

10. The hollow mesoporous silica rod according to claim 9, wherein the capping layer is formed of inorganic silica.

11. The hollow mesoporous silica rod according to claim 1, wherein the silica shell has an aspect ratio of 1:2 to 1:40.

12. A method for producing a hollow mesoporous silica rod, comprising the steps of:
preparing a rod-shaped inorganic silica core;
forming a mesoporous organic silica shell on the surface of the inorganic silica core to produce a core-shell silica rod; and
removing the inorganic silica core within the core-shell silica rod.

13. The method for producing a hollow mesoporous silica rod according to claim 12, wherein the step of preparing a rod-shaped inorganic silica core comprises the steps of:
preparing an oil-in-water emulsion comprising an alcoholic solvent and a surfactant; and
adding an inorganic silica precursor to the oil-in-water emulsion to grow the inorganic silica in a rod shape.

14. The method for producing a hollow mesoporous silica rod according to claim 12, wherein the step of preparing a rod-shaped inorganic silica core comprises the steps of:
preparing inorganic silica nanoseeds by adding a silica precursor to a solution comprising an alcoholic solvent and a silica-forming catalyst;
preparing an oil-in-water emulsion comprising the inorganic silica nanoseeds, an alcoholic solvent, and a surfactant; and
a step of adding an inorganic silica precursor to the oil-in-water emulsion to grow inorganic silica in a rod shape.

15. The method for producing a hollow mesoporous silica rod according to claim 12, wherein the mesoporous organic silica shell is formed by reacting the inorganic silica core with a surfactant and an organic silica precursor in a mixed solvent of water and an alcoholic solvent.

16. The method for producing a hollow mesoporous silica rod according to claim 12, wherein the step of removing the inorganic silica core comprises etching the core-shell silica rod with an alkaline solution.

17. The method for producing a hollow mesoporous silica rod according to claim 16, wherein the alkaline solution comprises an alkali metal hydroxide.

18. The method for producing a hollow mesoporous silica rod according to claim 16, wherein the reaction temperature of the alkaline solution is 50°C to 90°C.

19. A method for supporting an active substance, comprising the steps of:
mixing the hollow mesoporous silica rod according to claim 1 with an active substance; and
injecting the active substance into the hollow portion of the hollow mesoporous silica rod.

20. The method for supporting an active substance according to claim 19, wherein
the active substance comprises a hydrophobic substance, and
the step of injecting the active substance comprises forming an oil-in-water emulsion from a mixed solution of the hollow mesoporous silica rod and the active substance.

21. The method for supporting an active substance according to claim 19, wherein the method further comprises the step of removing any remaining active substance not injected into the hollow portion by centrifugation.

22. The method for supporting an active substance according to claim 19, wherein the method further comprises the step of capping mesoporous pores present in the shell of the silica rod into which the active substance has been injected.

23. A cosmetic composition comprising the hollow mesoporous silica rod according to claim 1, wherein the active substance has been supported within the hollow portion, as an active ingredient.

24. The cosmetic composition according to claim 23, wherein the cosmetic composition is used for whitening, ameliorating wrinkles, removing body hair, alleviating acne-prone skin, alleviating hair loss symptoms, enhancing elasticity, or moisturizing the skin.

25. A pharmaceutical composition comprising the hollow mesoporous silica rod according to claim 1, wherein the active substance has been supported within the hollow portion, as an active ingredient.
